# EUROPÄISCHE PATENTSCHRIFT

(11) **EP 2 628 490 B1**
(45) Veröffentlichungstag und Bekanntmachung des Hinweises auf die Patenterteilung: **02.04.2014**
(21) Anmeldenummer: 12000650.7
(22) Anmeldetag: 01.02.2012
(51) Int. Cl.: A61L 27/26, A61L 27/56, A61L 27/58

(54) **Matrix zur Zellbesiedelung**
Matrix for cell colonisation
Matrice de colonisation cellulaire

(43) Veröffentlichungstag der Anmeldung: 21.08.2013
(73) Patentinhaber: Bioenergy Capital AG, 50668 Köln (DE)
(72) Erfinder: Görne, Martin, DE-22337 Hamburg (DE); Kordick, Thomas, DE-63773 Goldbach (DE)
(74) Vertreter: Diehl & Partner GbR

(56) Entgegenhaltungen:
- EP-A1- 1 264 607
- EP-A1- 1 674 048
- WO-A1-2006/061229
- WO-A2-00/78928

## Beschreibung

Die vorliegende Anmeldung bezieht sich auf poröse Matrices zur Zellbesiedlung für therapeutische oder diagnostische Zwecke.

Zellimplantate auf der Basis poröser Matrices aus bioverträglichen Polymeren sind aus WO 2004/108810 A1 und EP 1264 607 A1 bekannt. Bei solchen Matrices sind die Poren vernetzt und dienen als Templat für die Ansiedlung von Zellen *in vivo* (z. B. therapeutisch) oder *in vitro* (z. B. diagnostisch). Bei Transplantationen kann eine solche bioresorbierbare Matrix zur temporären Lokalisation des Transplantats dienen.

Die bekannten Template sind bei einigen Anwendungen noch nicht voll befriedigend, insbesondere hinsichtlich der klinischen Ergebnisse.

Die Erfindung setzt sich daher zum Ziel, eine Verbesserung der klinischen Performance der Template zu erreichen.

Dazu schlägt die Erfindung vor, auf einer Seite des für gewöhnlich scheibenförmigen Templats eine Oberfläche mit weniger als 20% des durchschnittlichen Porenanteils der anderen Seite(n) bereitzustellen. Diese asymmetrische Struktur ermöglicht, das Templat so im Körper anzuordnen, dass die implantierten vitalen Zellen länger darin bleiben.

Unter einem weiteren Aspekt schlägt die Erfindung Verfahren zur Herstellung von porösen bioresorbierbaren Matrices vor, wobei initial eine Polymerschicht ohne Porenbildner gebildet wird, worauf dann ein Gemisch aus mindestens zwei Polymeren, einem Lösungsmittel für eines der Polymere und einem wasserlöslichen Porenbildner aufgeschichtet wird, gefolgt von Abdampfen des Lösungsmittels und dann Wässern zum Entfernen des Porenbildners. Zwischen diesen letzten beiden Schritten kann in einer Variante noch eine Beaufschlagung mit Druck zum Kompaktieren stattfinden. Beide Verfahren führen zu hochporösen Polymermatrixscheiben, deren eine Seite jedoch eine porenfreie oder zumindest porenarme Membran aufweist. Trotz dieser Membran ist die Versorgung der im Anwendungsfall in den Poren befindlichen Zellen ausreichend, die Verlustraten durch Abwanderung sind jedoch erheblich reduziert. Soll in einer Variante ein solches Templat als Bindegewebsunterstützung dienen, kann die durchgehende Polymerschicht genügend Halt für z. B. Nahtmaterial zur Fixierung des Templats bereitstellen.

Erfindungsgemäß ist die poröse Matrix hydrophil mit Acrylsäure-Polymerisat beschichtet. Dazu wird ein Plasma-Beschichtungsschritt gefolgt von einem plasmalosen Beschichtungsschritt, wodurch die benötigten Schichtdicken von über einem Mikrometer erreicht werden, wobei die initial erzeugte Teil-Schicht dünner ist als die danach plasmalos erzeugte Teil-Schicht.

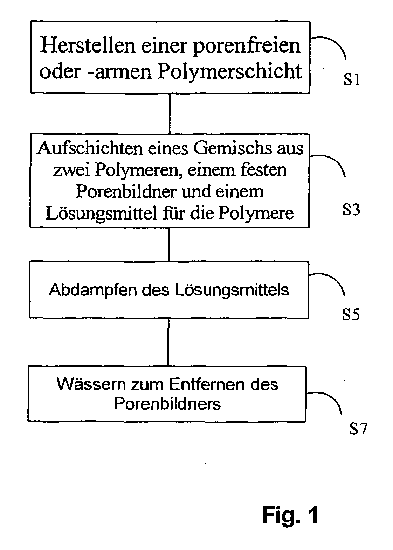

Weitere Merkmale der Erfindung ergeben sich aus der nachfolgenden Beschreibung von Ausführungsbeispielen in Verbindung mit den Ansprüchen sowie den Figuren. Die Erfindung ist nicht auf die beschriebenen Ausführungsbeispiele beschränkt, sondern durch den Umfang der beiliegenden Patentansprüche bestimmt. Insbesondere können die einzelnen Merkmale bei erfindungsgemäßen Ausführungsformen in anderer Anzahl und Kombination als bei den untenstehend angeführten Beispielen verwirklicht sein. Bei der nachfolgenden Erläuterung eines Ausführungsbeispiels wird auf die beiliegenden Figur Bezug genommen, die ein Flussdiagramm für ein erfindungsgemäßes Verfahren zeigt.

In einer Hauptanwendung werden Matrices zur Besiedelung mit Funktionszellen bereitgestellt, beispielsweise mit Hepatozyten und/oder mit Langerhans'schen Inselzellen. Solche biochemischen Funktionszellen heften sich an die Innenwände der Poren der schaumartigen Matrix an (Anheftungsraten über 80% oder, geeignet beschichtet, über 95%) und können mit der Matrix in mesotheliale Taschen transplantiert werden, idealerweise des Zellspenders selbst. Dabei wird ausgenutzt, dass in diesem Fall keine Abstoßungsreaktion erfolgt, sondern nur ein vergleichsweise milder, für den therapeutischen Prozess günstiger Fremdkörper-Reiz ausgeübt wird. Binnen weniger Wochen wird die Matrix vaskularisiert und sind die implantierten Zellen nicht mehr nur auf diffusive Versorgung angewiesen. Die Matrices werden so angeordnet, dass die porenarme (oder -freie) Seite innen und die porenreiche Seite außen liegt, um die Verlustrate durch Abwanderung der Zellen niedrig zu halten. Parallel findet eine allmähliche Auflösung eines Teils der abbaubaren Matrix (binnen 3-4 Monaten, oder mindestens 2 und/oder weniger als 7 Monaten) statt und wird das physiologische Milieu dadurch in einer Weise beeinflusst, die ebenfalls dem therapeutischen Erfolg dienlich ist. Es ist zweckmäßig, wenn ein Teil der Polymermischung langsamer erodiert (Verhältnis der Abbauzeiten mindestens 5) und den strukturellen Zusammenhalt längere Zeit, z. B. 2,5-3 Jahre (oder mindestens 2 und/oder weniger als 5 Jahre) gewährleistet. Solche Polymere sind zweckmäßig auf Basis von α-Hydroxycarbonsäuren wie Milchsäure und/oder Glykolsäure, z. B. PLA oder PLGA. Die Hersteller solcher zum Einsatz im menschlichen Körper zugelassener Polymere geben die hier relevanten nominellen Abbauzeiten an.

Wie bereits erwähnt, werden besonders gute Anheftungsraten bei beschichteten Matrices beobachtet, nämlich solchen, die in einem kombinierten PECVD/CVD-Verfahren zunächst mit einer dünnen (z. B. 20-30 nm) PAA-Lage plasmabeschichtet und daran anschließend mit einer dickeren PAA-Lage (z. B. 20-30 µm) ohne Plasma-Einwirkung beschichtet werden. Diese obere Lage bildet eine kristalline, hydrophile Schicht aus. Zunächst wird eine Lösung eines der verwendeten Polymere in für medizinische Zwecke zugelassenem Chloroform in eine Form gegossen und das Lösungsmittel bei 45°-65° abgedampft. Daraufhin wird eine Polymer-Mischung definierter Partikelgrößen-Verteilung mit einem Kochsalz-Granulat ebenfalls definierter Partikelgrößen-Verteilung gemischt, mit einer Lösung eines der Polymere in Chloroform vermengt und dann auf die bereits hergestellte Polymerschicht gegeben. Aus diesem Vorformling dampft das Lösungsmittel bei leicht erhöhter Temperatur (45°C-65°C) ab und dieser kann dann gewünschtenfalls durch Beaufschlagung mit Druck kompaktiert werden. Anschließend wird der Kompaktkörper gewässert, um durch Entfernen des Salzes die gewünschte Porosität bereitzustellen. Dabei bleibt aber die initial hergestellte Polymerschicht porenfrei. Je nach Einsatzzweck kann die Dicke der porenarmen Schicht durch Menge und Konzentration der anfänglichen Lösung eingestellt werden. Beispielsweise erhält man eine sehr dünne Membran, wenn die Konzentration der Lösung niedrig ist (z. B. 4 %ig in Chloroform, langsam abbaubares Polymer) und die Füllhöhe klein (z. B. 0,1-1 mm, beispielsweise 0,3 mm). Wird eine mechanisch stärker belastbare Struktur gewünscht, kann bei gleicher Konzentration höher (z. B. 5-50 mm, typisch 20-25 mm) eingefüllt werden. Das Abdampfen des Chloroforms dauert dann entsprechend länger (1,5 h). Im ersten Fall hat die entstehende Membran eine Dicke von ca. 10-20 µm, im letzteren Fall von ca. 0,5-1 mm.

Die Kochsalzpartikel der aufzuschichtenden Mischung sind etwas gröber (Median bei 350-370 µm) als die Polymerpartikel (Median des langsamer abbaubaren Polymers zwischen 210 µm und 230 µm, der des schneller abbaubaren Polymers zwischen 150 µm und 170 µm). Dabei sind die Verteilungsbreiten (5 %/95 %) ähnlich, nämlich etwa ± 85-95 µm für Salz bzw. Polymer insgesamt. Die Verteilungsform kann bi-oder trimodal sein. Die Zusammensetzung der Schichtmischung ist zu etwa 96 % Salz, 1-1,5 % festes Polymer und weitere ca. 3-5 % gelöstes Polymer, wobei die Volumenanteile von Feststoffen und Flüssigkeit etwa gleich sind. Insgesamt beträgt der Anteil des schnell abbaubaren Polymers lediglich etwa 5-20 % des Polymers. Die Gesamtdicke der porenbildenden Schicht ist 5-6 mm. In der Variante einer stabileren Initialschicht kann das Salz etwas gröber gewählt werden (Median ca. 400-420 µm). In diesem Fall ist die Gesamtdicke der porenbildenden Schicht 4-5 mm und kann auf das Kompaktieren mit Druck verzichtet werden. Das Wässern dauert ca. 24 h und wird gefolgt von einer Trocknung bei 45-50°C. Wenn eine Beschichtung vorgenommen wird, liegt die Matrix mit der porenarmen Seite auf und wird daher überwiegend die offenporige Seite beschichtet.

Die hier verwendeten Polymere sind z. B. von der Fa. Evonik erhältlich und tragen die Bezeichnungen L210s, L210, L09s, L207s, L206s (langsamer abbaubare PLGA-Polymere) bzw. RG502, RG502H, RG505 (schneller abbaubare PLGA-Polymere).

Bei einer Verwendung außerhalb des Körpers kann eine Matrix gemäß der obigen Beschreibung zur Fixierung von Zellen dienen, die in einem Bioreaktor Agenzien ausgesetzt werden. Beispielsweise können definierte Zelltypen auf diese Weise daraufhin untersucht werden, ob sie auf in Frage kommende Medikamente ansprechen oder nicht, und kann die Therapie in Abhängigkeit von derart erhaltenen Untersuchungsergebnissen geplant werden. Ebenso kann die Medikamentenentwicklung vereinfacht werden, weil Toxizität frühzeitig erkannt wird.

## Patentansprüche

1. Zellimplantat-Matrix mit einer konnektiven Porosität von über 80 %, überwiegend bestehend aus einem Gemisch bioresorbierbarer Polymere, wobei die Matrix Scheibenform aufweist und wobei die Oberfläche auf einer Seite der Scheibe weniger als 20 % des durchschnittlichen Porenanteils der anderen Seiten aufweist, wobei die Matrix mit Polyacrylsäure, PAA, hydrophilierend beschichtet ist, wobei eine initial mit Einwirkung eines Plasmas erzeugte PAA-Teil-Schicht dünner ist als eine danach ohne Plasma-Einwirkung erzeugte PAA-Teil-Schicht.

2. Matrix nach Anspruch 1, wobei die Beschichtung überwiegend aus Acrylsäureeinheiten besteht.

3. Matrix nach Anspruch 1 oder 2, wobei die Dicke der Beschichtung mehr als 1 µm beträgt.

4. Matrix nach einem der vorstehenden Ansprüche, wobei die Matrix überwiegend aus Poly(α-hydroxy)carbonsäuren besteht.

5. Matrix nach einem der vorstehenden Ansprüche, wobei sich Resorptionsraten zweier der das Gemisch bildenden Polymere, die jeweils mindestens 10 % des Gemischs ausmachen, um einen Faktor von mehr als 5 unterscheiden.

6. Verfahren zur Herstellung einer porösen Zellimplantat-Matrix, das Verfahren umfassend:
Herstellen einer bioresorbierbaren Polymerschicht;
Aufschichten eines Gemisches aus einem wasserlöslichen Feststoff, mindestens zwei Polymeren mit verschiedenen Resorptionsraten und einem mit Wasser nicht mischbaren Lösungsmittel für wenigstens eines der Polymere auf die Polymerschicht;
Abdampfen des Lösungsmittels;
Wässern des Agglomerats zum Entfernen des wasserlöslichen Feststoffs; und
Beschichten mit einer hydrophilierenden Komponente nach dem Wässern, wobei sich an einen initialen PAA-Plasma-beschichtungsschritt in Gegenwart eines Edel- oder Inertgases ein PAA-Beschichtungsschritt ohne Plasma derart anschließt, dass die ohne Plasma erzeugte Schichtdicke größer ist als die initial erzeugte Schichtdicke.

7. Verfahren nach Anspruch 6, ferner umfassend Beaufschlagen mit Druck zum Kompaktieren des Gemisches nach dem Abdampfen des Lösungsmittels.

8. Verfahren nach Anspruch 6 oder 7, wobei als Lösungsmittel Chloroform verwendet wird.

9. Verfahren nach einem der Ansprüche 6 bis 8, wobei sich Resorptionsraten zweier der das Gemisch bildenden Polymere, die jeweils mindestens 10 % des Gemischs ausmachen, um einen Faktor von mehr als 5 unterscheiden.

10. Verfahren nach einem der Ansprüche 6 bis 9, wobei die hydrophilierende Komponente überwiegend aus Acrylsäure oder Acrylsäureanhydrid besteht.

11. Verfahren nach einem der Ansprüche 6 bis 10, wobei die Dicke der initial hergestellten Schicht 0,002-2,5 mm beträgt.

12. Verfahren nach Anspruch 11, wobei die Dicke der initial hergestellten Schicht 0,005-0,025 mm beträgt.

13. Verfahren nach Anspruch 11, wobei die Dicke der initial hergestellten Schicht 0,25-2,0 mm beträgt.

14. Verwendung der Matrix nach einem der Ansprüche 1 bis 5 zur Besiedelung mit vitalen Zellen und zum Aussetzen der vitalen Zellen einem vorbestimmten Test-Agens außerhalb des Körpers.

15. Verwendung der nach dem Verfahren gemäß einem der Ansprüche 6 bis 13 hergestellten Matrix zur Besiedelung mit vitalen Zellen und zum Aussetzen der vitalen Zellen einem vorbestimmten Test-Agens außerhalb des Körpers.

## Claims

1. A matrix for cell implants having a connective porosity of more than 80 %, mainly consisting of a mixture of bioresorbable polymers, wherein the matrix has disk shape, and wherein the surface on one side of the disk has less than 20 % of the average pore proportion of the other sides, wherein the matrix is hydrophilizingly coated with poly(acrylic acid), PAA, wherein a partial PAA layer initially generated under the influence of a plasma is thinner than a partial PAA layer subsequently generated without plasma action.

2. The matrix of claim 1, wherein the coating consist mainly of acrylic acid units.

3. The matrix of claim 1 or 2, wherein the thickness of the coating is more than 1 µm.

4. The matrix of one of the preceding claims, wherein the matrix consists mainly of poly((α-hydroxy)carbonic acids).

5. The matrix of one of the preceding claims, wherein resorption rates of two of the polymers constituting the mixture, each accounting for at least 10% of the mixture, differ by a factor of at least 5.

6. A process of manufacturing a porous matrix for cell implants, the process comprising:
manufacturing a bioresorbable polymer layer;
stratifying a mixture of a water soluble solid, at least two polymers with different resorption rates, and a non-water-miscible solvent for at least one of the polymers onto the polymer layer;
evaporating the solvent;
watering the agglomerate to remove the water soluble solid; and
after the watering, coating with a hydrophilizing component, wherein an initial PAA-plasma coating step in the presence of a noble or inert gas is followed by a PAA coating step without plasma such that a layer thickness generated without plasma is larger than the initially generated layer thickness.

7. The process of claim 6, further comprising application of pressure for compacting the mixture after evaporating the solvent.

8. The process of claim 6 or 7, wherein chloroform is used as the solvent.

9. The process of one of claims 6 to 8, wherein resorption rates of two of the polymers constituting the mixture, each accounting for at least 10 % of the mixture, differ by a factor of at least 5.

10. The process of one of claims 6 to 9, wherein the hydrophilizing component consists mainly of acrylic acid or acrylic acid anhydride.

11. The process of one of claims 6 to 10, wherein the thickness of the initially manufactured layer is 0.002-2.5 mm.

12. The process of claim 11, wherein the thickness of the initially manufactured layer is 0.005-0.025 mm.

13. The process of claim 11, wherein the thickness of the initially manufactured layer is 0.25-2.0 mm.

14. Use of the matrix of one of claims 1 to 5 for infiltration with viable cells and for exposing the viable cells to a predefined test agent outside the body.

15. Use of the matrix manufactured according to one of claims 6 to 13 for infiltration with viable cells and for exposing the viable cells to a predefined test agent outside the body.

## Revendications

1. Matrice d'implant cellulaire présentant une porosité connective supérieure à 80 %, consistant principalement en un mélange de polymères biorésorbables, la matrice présentant la forme d'un disque et la surface sur un côté du disque présente moins de 20 % de la proportion de pores moyenne des autres côtés, la matrice étant revêtue d'acide polyacrylique, PAA, hydrophilisant, dans laquelle une couche partielle de PAA produite initialement sous l'effet d'un plasma est plus fine qu'une couche de PAA produite ensuite sans l'effet d'un plasma.

2. Matrice selon la revendication 1, dans laquelle le revêtement consiste principalement en motifs d'acide acrylique.

3. Matrice selon la revendication 1 ou 2, dans laquelle l'épaisseur du revêtement est supérieure à 1 µm.

4. Matrice selon l'une des revendications précédentes, la matrice consistant principalement en acides poly(α-hydroxy)carboxyliques.

5. Matrice selon l'une des revendications précédentes, dans laquelle des taux de résorption de deux des polymères formant le mélange qui constituent respectivement au moins 10 % du mélange se distinguent d'un facteur supérieur à 5.

6. Procédé de production d'une matrice d'implant cellulaire poreuse, le procédé comprenant :
la production d'une couche polymère biorésorbable ;
l'application d'un mélange d'un solide soluble dans l'eau, d'au moins deux polymères présentant des taux de résorption différents et d'un solvant non miscible dans l'eau pour au moins l'un des polymères, sur la couche polymère ;
l'évaporation du solvant ;
le lavage de l'agglomérat pour éliminer le solide soluble dans l'eau ; et
le revêtement avec un composant hydrophilisant après le lavage, dans lequel une étape de revêtement initiale de PAA au plasma en présence d'un gaz rare ou d'un gaz inerte se joint à une étape de revêtement de PAA sans plasma de telle sorte que l'épaisseur de couche produite sans plasma soit supérieure à l'épaisseur de couche produite initialement.

7. Procédé selon la revendication 6, comprenant en outre l'application de pression pour compacter le mélange après l'évaporation du solvant.

8. Procédé selon la revendication 6 ou 7, dans lequel on utilise comme solvant, du chloroforme.

9. Procédé selon l'une des revendications 6 à 8, dans lequel des taux de résorption de deux des polymères formant le mélange, qui constituent respectivement au moins 10 % du mélange, se distinguent d'un facteur supérieur à 5.

10. Procédé selon l'une des revendications 6 à 9, dans lequel les composants hydrophilisants consistent principalement en acide acrylique ou en anhydride d'acide acrylique.

11. Procédé selon l'une des revendications 6 à 10, dans lequel l'épaisseur de la couche produite initialement est de 0,002 à 2,5 mm.

12. Procédé selon la revendication 11, dans lequel l'épaisseur de la couche produite initialement est de 0,005 à 0,025 mm.

13. Procédé selon la revendication 11, dans lequel l'épaisseur de la couche produite initialement est de 0,25 à 2,0 mm.

14. Utilisation de la matrice selon l'une des revendications 1 à 5, pour la colonisation avec des cellules vitales et pour l'exposition des cellules vitales à un agent de test prédéterminé en dehors du corps.

15. Utilisation de la matrice produite selon le procédé selon l'une des revendications 6 à 13, pour la colonisation avec des cellules vitales et pour l'exposition des cellules vitales à un agent de test prédéterminé en dehors du corps.
